# EUROPEAN PATENT APPLICATION

(11) **EP 3 757 092 A1**
(43) Date of publication of application: **30.12.2020**
(21) Application number: 18907100.4
(22) Date of filing: 20.09.2018
(51) Int. Cl.: C07D 233/90

(54) **METHOD FOR PRODUCING 4,5-DICYANO-2-(FLUOROALKYL)IMIDAZOLE**

(30) Priority: 23.02.2018 JP 2018030980
(71) Applicant: Nippon Soda Co., Ltd., Tokyo 100-8165 (JP)
(72) Inventor: MATSUKIDA, Seiji, Odawara-shi, Kanagawa 250-0280 (JP); KOIZUMI, Satoshi, Odawara-shi, Kanagawa 250-0280 (JP); INOUE, Tsutomu, Odawara-shi, Kanagawa 250-0280 (JP)
(74) Representative: Mewburn Ellis LLP
(86) International application number: PCT/JP2018/034851
(87) International publication number: WO 2019/163178

(57) **Abstract**

It is an object of the present invention to provide a method for synthesizing a 4,5-dicyano-2-(fluoroalkyl)imidazole using diaminomaleonitrile (DAMN) as a starting material, wherein the 4,5-dicyano-2-(fluoroalkyl)imidazole may be synthesized in a high yield without requiring any complicated operations. The method for synthesizing the 4,5-dicyano-2-(fluoroalkyl)imidazole of the present invention comprises reacting a compound of formula (ii): R_{f}-COOH (ii) (wherein R_{f} is a C1-C10 fluoroalkyl group or a C3-C10 fluorocycloalkyl group) or a salt thereof with a compound of formula (iii): X-R (iii) (wherein X is Cl, Br or I, and R is an unsubstituted or substituted C1-C6 alkylsulfonyl group or an unsubstituted or substituted phenylsulfonyl group) and diaminomaleonitrile in a solvent in the presence or absence of a base.

## Description

### Technical Field

The present invention relates to a method for producing a 4,5-dicyano-2-(fluoroalkyl)imidazole.

The present application claims priority to Japanese Patent Application No. 2018-30980 filed on February 23, 2018, and the contents thereof are incorporated herein by reference.

### Background Art

Lithium salts are used as electrolytes of lithium ion batteries. Of the lithium salts, lithium hexafluorophosphate (LiPF₆) is most generally used, but it has a problem of safety because it has a defect of being decomposed into a form of hydrogen fluoride gas. On that account, a lithium salt of 2-fluoroalkyl-4,5-dicyanoimidazole, such as lithium 2-trifluoromethyl-4,5-dicyanoimidazolate (LiTDI) and lithium 2-pentafluoroethyl-4,5-dicyanoimidazolate (LiPDI), has been developed.

As a method for synthesizing a 2-fluoroalkyl-4,5-dicyanoimidazole that is an intermediate of this lithium salt, synthesis thereof from diaminomaleonitrile (DAMN) is known.

In Patent Document 1, a method including (a) production (step 1) of an amide compound from diaminomaleonitrile and a fluoro compound RfCOY [wherein Y represents a chlorine atom or an OCORf group] at a temperature T₁ and (b) formation (step 2) of an imidazole compound [wherein Rf represents a C1-5 fluoroalkyl group] from the amide compound through cyclodehydration at a temperature T₂ that is higher than T₁ is described.

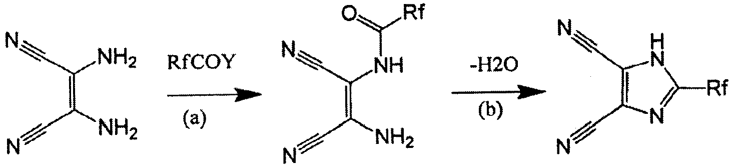

In the method of Patent Document 1, however, when the fluoro compound RfCOY is an acid anhydride (when Y is an OCORf group), a perfluoroalkanecarboxylic acid is produced as a by-product. Since this acid is a strongly acidic and strongly corrosive substance, complicated sub-steps for removing this from the reaction system and treating the acid are necessary.

When the fluoro compound RfCOY is a chloride (when Y is a chlorine atom), it has a problem that it is hard to use as a raw material because RfCOCl is a gas at ordinary temperature and normal pressure in many cases and has strong toxicity and corrosiveness.

In Patent Document 1, WO2010/023413 (corresponding republished publication: Patent Document 2) is given as a background art document. It is described in Patent Document 1 that according to the method as described in Patent Document 2, namely, the method for synthesizing an imidazole compound from diaminomaleonitrile and a fluoro compound RfCOY in one step, the final yield of a lithium salt finally obtained is about 70%, and the impurities need a severe purification step, so that the method is unsuitable for industrialization of a lithium salt.

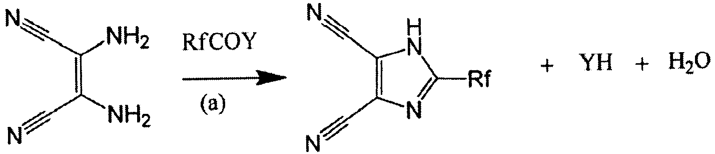

### [Y represents OCORf, Cl, F, CF₃SO₃, OCH₃, OC₂H₅, OCH₂CF₃, OC₆H₄NO₂, an imidazolyl group or a succinimidyloxy group]

In Patent Document 3, a method for reacting diaminomaleonitrile with trifluoroacetate is also described as a method similar to the above.

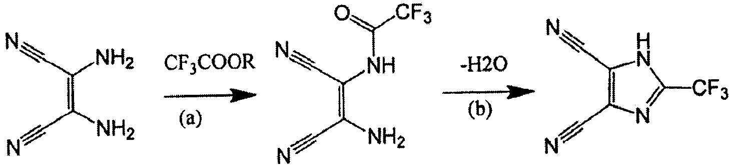

Here, as R, methyl, ethyl, butyl and cyclohexyl are exemplified.

Also in this method, in order to improve the yield, two steps of amidation and cyclodehydration are required similarly to the method of Patent Document 1, and operations of, for example, carrying out the reaction at two-step reaction temperatures are required.

On the other hand, as a synthesis method related to the present invention, a method for synthesizing an amide compound by allowing a sulfonic acid halide as an activator to act on a carboxylic acid to synthesize an active ester and reacting an amine or its derivative with the active ester, as shown by the following reaction formula, is known (Patent Document 4).

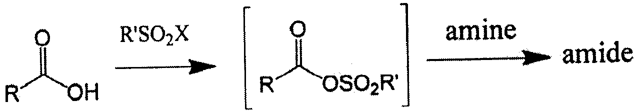

It is described in Patent Document 4 that an aromatic amine, an alkylamine, a primary amine or a secondary amine may be applied as the amine, but specifically, an example about aniline is only shown, and whether such a substance may be applied or not to cyclization reaction of a compound having a cyano group and having two or more amino groups, such as diaminomaleonitrile, is not suggested in the document, and additionally, occurrence of many side reactions is presumed. Therefore, whether the method may be practically applied or not is unknown.

### Prior Art Documents

### Patent Documents

Patent Document 1: Japanese unexamined Patent Application Publication (Translation of PCT Application) No. 2014-533255
Patent Document 2: Japanese unexamined Patent Application Publication (Translation of PCT Application) No. 2012-500833
Patent Document 3: Chinese unexamined Patent Application Publication No. 106008262
Patent Document 4: Japanese unexamined Patent Application Publication No. 2016-37476

### Summary of the Invention

### Object to be Solved by the Invention

It is an object of the present invention to provide a method for synthesizing a 4,5-dicyano-2-(fluoroalkyl)imidazole using diaminomaleonitrile (DAMN) as a starting material, wherein the 4,5-dicyano-2-(fluoroalkyl)imidazole may be synthesized in a high yield without requiring any complicated operations.

### Means to Solve the Object

As a result of earnest studies, the present inventors have found that by reacting a fluorocarboxylic acid or a salt thereof and a sulfonic acid halide with diaminomaleonitrile, a 4,5-dicyano-2-(fluoroalkyl)imidazole may be synthesized in one step in a high yield, and they have accomplished the present invention.

That is to say, the present invention relates to the following inventions.
(1) A method for producing a 4,5-dicyano-2-(fluoroalkyl)imidazole of formula (i): 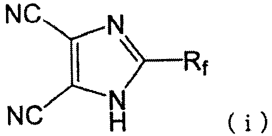
   (wherein R_{f} is a C1-10 fluoroalkyl group or a C3-10 fluorocycloalkyl group), comprising reacting a compound of formula (ii):

      R_{f}-COOH (ii)
   (wherein R_{f} is the same as in formula (i)) or a salt thereof with a compound of formula (iii):

      X-R (iii)
   (wherein X is Cl, Br or I, and R is an unsubstituted or substituted C1-6 alkylsulfonyl group or an unsubstituted or substituted phenylsulfonyl group) and diaminomaleonitrile in a solvent in the presence or absence of a base.
(2) The method for producing the 4,5-dicyano-2-(fluoroalkyl)imidazole according to "1", wherein after the compound of formula (ii) and diaminomaleonitrile are added, the compound of formula (iii) is added.
(3) The method for producing the 4,5-dicyano-2-(fluoroalkyl)imidazole according to "1" or "2", wherein the compound of formula (ii) is trifluoroacetic acid.
(4) The method for producing the 4,5-dicyano-2-(fluoroalkyl)imidazole according to any one of "1" to "3", wherein the compound of formula (iii) is methanesulfonyl chloride.

### Effect of the Invention

By reacting a fluorocarboxylic acid or a salt thereof and a sulfonic acid halide with diaminomaleonitrile, a 4,5-dicyano-2-(fluoroalkyl)imidazole may be synthesized in one step in a high yield (yield > 90%) (see Examples 1 and 2).

In a method in which a fluorocarboxylic acid or a salt thereof is reacted with a sulfonic acid halide first to synthesize an active ester compound and then the active ester compound is reacted with diaminomaleonitrile, the yield is lowered even if the same raw materials are used (see Comparative Examples 1 and 2, yield: about 60%).

Even when a chloroformic acid ester (note) is used instead of the sulfonic acid halide and reacted in the same manner as in the synthesis method of the present invention, the desired compound is hardly obtained (see Comparative Example 3, yield: 6%).

Note) It is thought that a chloroformic acid ester reacts with a fluorocarboxylic acid to produce the same active ester compound as previously described (see Chem. Pham. Bull, 40, 396).

### Mode of Carrying Out the Invention

### (Reaction starting material)

The method for producing the 4,5-dicyano-2-(fluoroalkyl)imidazole of the present invention is characterized by reacting a compound of formula (ii):

R_{f}-COOH (ii)

or a salt thereof with a compound of formula (iii):

X-R (iii)

and diaminomaleonitrile.

In the above reaction formula, R_{f} of each of formulae (i) and (ii) represents a C1-10 fluoroalkyl group or a C3-C10 fluorocycloalkyl group.

The C1-C10 fluoroalkyl group is a group in which all or a part of hydrogen atoms of a linear or branched C1-C10 alkyl group have been substituted by F atoms, and CF₃, CHF₂, CH₂F, C₂HF₄, C₂H₂F₃, C₂H₃F₂, C₂F₅, C₃F₇, C₃H₂F₅, C₃H₄F₃, C₄F₉, C₄H₂F₇, C₄H₄F₅, C₅F₁₁, C₆F₁₃, C₇F₁₅, C₈F₁₇, C₁₀F₂₁ or the like may be exemplified.

The C3-C10 fluorocycloalkyl group is a group in which all or a part of hydrogen atoms of a cyclic C3-C10 alkyl group have been substituted by F atoms, and C₃F₅, C₃H₄F, C₃HF₄, C₄F₇, C₄H₄F₃, C₄HF₆, C₅F₉, C₆F₁₁, C₇F₁₃, C₈H₁₅, C₁₀F₁₉ or the like may be exemplified.

X in formula (iii) is Cl, Br or I.

R in formula (iii) represents an optionally substituted C1-C6 alkylsulfonyl group or an optionally substituted phenylsulfonyl group.

As the C1-C6 alkylsulfonyl group, a methanesulfonyl group, an ethanesulfonyl group, a propanesulfonyl group, a butanesulfonyl group, a pentanesulfonyl group, or a hexanesulfonyl group may be exemplified.

The C1-C6 alkylsulfonyl group and the phenylsulfonyl group may be each substituted by an alkyl group, an alkoxy group, a halogen atom, a substituted amino group, an aryl group, a heteroaryl group, an aralkyl group or the like.

As the alkyl group, a linear, branched or cyclic alkyl group is exemplified, and examples thereof include a methyl group, an ethyl group, an n-propyl group, an isopropyl group, an n-butyl group, an isobutyl group, an s-butyl group, a t-butyl group, an n-pentyl group, an n-hexyl group, a cyclopropyl group, a cyclopentyl group and a cyclohexyl group.

As the alkoxy group that is a substituent, an alkoxy group containing a linear, branched or cyclic alkyl group having 1 to 6 carbon atoms is exemplified, and examples thereof include a methoxy group, an ethoxy group, an n-propoxy group, an isopropoxy group, an n-butoxy group, an isobutoxy group, an s-butoxy group, a t-butoxy group, a n-pentyloxy group, a n-hexyloxy group, a cyclopentyloxy group and a cyclohexyloxy group.

As the halogen atom, F, Cl, Br or I is exemplified.

As the substituted amino group, a mono or dialkylamino group, such as an N-methylamino group, an N,N-dimethylamino group, an N,N-diethylamino group, an N,N-diisopropylamino group or an N-cyclohexylamino group; a mono or diarylamino group, such as an N-phenylamino group, an N,N-diphenylamino group, an N-naphthylamino group or an N-naphthyl-N-phenylamino group; a mono or diaralkylamino group, such as an N-benzylamino group or an N,N-dibenzylamino group; or the like is exemplified.

As the aryl group, a phenyl group, a naphthyl group, a biphenyl group or the like is exemplified, and these aryl groups may be each substituted by such an alkyl group, an alkoxy group, a halogen atom, an amino group or the like as previously described.

As the heteroaryl group, a 5- to 8-membered monocyclic heteroaryl group or a polycyclic or condensed ring heteroaryl group, each containing, as heteroatoms, at least one to four heteroatoms, such as a nitrogen atom, an oxygen atom or a sulfur atom, is exemplified. Specific examples thereof include a furyl group, a thienyl group, a pyridyl group, a pyrimidinyl group, a pyrazinyl group, a pyridazinyl group, a pyrazolyl group, an imidazolyl group, an oxazolyl group, a thiazolyl group, a benzofuryl group, a benzothienyl group, a quinolyl group, an isoquinolyl group, a quinoxalinyl group, a phthalazinyl group, a quinazolinyl group, a naphthylidinyl group, a cinnolinyl group, a benzimidazolyl group, a benzoxazolyl group and a benzothiazolyl group.

As the aralkyl group, a benzyl group, a 1-phenethyl group or the like is exemplified.

As the compound of formula (ii), difluoroacetic acid, trifluoroacetic acid, 3,3,3-trifluoropropionic acid, 2, 2, 3, 3, 3-pentafluoropropionic acid, heptafluorolactic acid, undecafluorohexanoic acid, pentadecafluorooctanoic acid or the like is exemplified, and trifluoroacetic acid is preferable.

As the compound of formula (iii), methanesulfonyl chloride, chloromethanesulfonyl chloride, trifluoromethanesulfonyl chloride, paratoluenesulfonyl chloride or the like is exemplified, and methanesulfonyl chloride is preferable.

As the salt of the compound of formula (ii), a salt of an alkali metal such as Li, Na, K or Cs, or a salt of an alkaline earth metal such as Mg or Ca is exemplified.

### (Reaction conditions)

Diaminomaleonitrile, a fluorocarboxylic acid and a sulfonic acid halide that are raw materials are reacted with one another at the same time in a solvent in the presence or absence of a base.

Since the aminomaleonitrile, the fluorocarboxylic acid and the sulfonic acid halide may be reacted at the same time, one step is enough.

It is preferable that to a solvent, the diaminomaleonitrile and the fluorocarboxylic acid be added first and the sulfonic acid halide be finally added.

It is undesirable that the fluorocarboxylic acid be reacted with the sulfonyl acid halide in advance and then the reaction product be reacted with the diaminomaleonitrile, because the yield is lowered.

As the base, an inorganic base or an organic base may be used. As the inorganic base, a carbonate, such as sodium carbonate or potassium carbonate; a hydroxide, such as sodium hydroxide or potassium hydroxide; any of alkoxides, such as sodium methoxide, sodium ethoxide or potassium tert-butoxide; a lithium salt, such as n-butyllithium, tert-butyllithium or lithium diisopropylamide; ammonia; or the like is exemplified. As the organic base, any of alkylamines, such as trimethylamine, triethylamine or diisopropylethylamine; any of heteroaryls, such as pyridine or picoline; any of arylamines, such as aniline or toluidine; any of amidines, such as diazabicyclononene or diazabicycloundecene; or the like is exemplified.

As the base for use in the present reaction, an organic base or ammonia is preferable, and as the organic base, any of alkylamines is more preferable.

As the solvent, an aprotic polar solvent or a nonpolar solvent may be used.

As the aprotic polar solvent, any of amides, such as acetone, methyl ethyl ketone, methyl isobutyl ketone, cyclohexanone, N,N-dimethylformamide, N,N-dimethylacetamide, N-methylpyrrolidone, 1,3-dimethyl-2-imidazolidinone or hexamethylphosphoric acid phosphoramide; any of ethers, such as diethyl ether, tetrahydrofuran, dioxane or 1,2-dimethoxyethane; any of nitriles, such as acetonitrile, propionitrile, butyronitrile or benzonitrile; dimethyl sulfoxide, sulfolane, or the like is exemplified.

As the nonpolar solvent, any of aromatic hydrocarbons, such as benzene, toluene, xylene, chlorobenzene, bromobenzene or dichlorobenzene; any of aliphatic hydrocarbons, such as n-pentane, n-hexane, n-heptane, n-octane, n-nonane, n-decane or ISOPAR G; any of alicyclic hydrocarbons, such as cyclopentane, cyclohexane or cyclooctane; or the like is exemplified.

These solvents may be used alone or used by combination of two or more thereof.

As the solvent for use in the present reaction, an aprotic polar solvent is preferable, and any of amides or any of nitriles is more preferable.

Regarding the amount of each raw material used, the amount of the fluorocarboxylic acid is 0.5 to 2.0 mol, and preferably 0.7 to 1.5 mol, and the amount of the sulfonic acid halide is 0.4 to 4.0 mol, and preferably 0.5 to 2.5 mol, with respect to 1 mol of the diaminomaleonitrile, but in usual, the fluorocarboxylic acid and the sulfonic acid halide are each used in an amount of 1 mol or more.

The amount of the base is 0.3 to 5.0 mol, and preferably 0.4 to 3.0 mol, with respect to 1 mol of the diaminomaleonitrile.

The reaction is carried out in the temperature range of room temperature to a boiling point of the solvent used. The reaction time is usually in the range of 1 to 48 hours, and preferably in the range of 1 to 21 hours. The reaction may be carried out at normal pressure, and from the viewpoint that a special reaction vessel, such as a pressure device, does not need to be used, the reaction is industrially advantageous. After the reaction, the solvent is distilled away, and if necessary, purification step such as recrystallization is carried out, whereby the desired compound may be produced in a high yield.

### Examples

Examples of synthesis of 4,5-dicyano-2-(trifluoromethyl)imidazole will be explained below, but the technical scope of the present invention is not intended to be limited to these examples.

The measurement conditions of HPLC used in the measurement of products are as follows.
Column: Phenomenex GeminiNX 4.6 × 250 mm
Eluent: acetonitrile: 0.1M K2HPO4 = 20:80
Flow rate: 1 ml/min
Measurement wavelength: 254 nm
Temperature: 40°C
Compound 1 retention time: 18 min

### Example 1

### (Method including adding trifluoroacetic acid and 2,3-diaminomaleonitrile to a solvent and then adding methanesulfonyl chloride thereto to react them; solvent: N-methylpyrrolidone)

To N-methylpyrrolidone (24.5 ml), trifluoroacetic acid (0.45 ml, 0.67 g, 5.88 mmol), triethylamine (0.75 ml, 0.54 g, 5.38 mmol) and 2,3-diaminomaleonitrile (0.53 g, 4.90 mmol) were added, and immediately thereafter, they were heated in an oil bath at 129°C. Under that temperature condition, methanesulfonyl chloride (0.42 ml, 0.62 g, 5.42 mmol) having been dissolved in N-methylpyrrolidone (4.9 ml) was dropwise added (0.5 hour), and the resulting solution was continuously stirred for 18 hours, then cooled to room temperature and analyzed by HPLC. The production ratio of 4,5-dicyano-2-(trifluoromethyl)imidazole was 92%.

### Example 2

### (Method including adding trifluoroacetic acid and 2,3-diaminomaleonitrile to a solvent and then adding methanesulfonyl chloride thereto to react them; solvent: propionitrile)

To propionitrile (24.5 ml), trifluoroacetic acid (0.45 ml, 0.67 g, 5.88 mmol), triethylamine (0.75 ml, 0.54 g, 5.38 mmol) and 2,3-diaminomaleonitrile (0.53 g, 4.90 mmol) were added, and immediately thereafter, they were heated in an oil bath at 96°C. Under that temperature condition, methanesulfonyl chloride (0.42 ml, 0.62 g, 5.42 mmol) having been dissolved in N-methylpyrrolidone (4.9 ml) was dropwise added (0.5 hour), and the resulting solution was refluxed while continuously stirring it for 21 hours, then cooled to room temperature and analyzed by HPLC. The production ratio of 4,5-dicyano-2-(trifluoromethyl)imidazole was 94%.

### Example 3

### (Method including adding trifluoroacetic acid and 2,3-diaminomaleonitrile to a solvent and then adding methanesulfonyl chloride thereto to react them; solvent: propionitrile)

To a 100 mL three-neck flask, 2,3-diaminomaleonitrile (3.0 g, 27.8 mmol) was added, then 14 mL of propionitrile was added, and they were stirred at 0°C while ice cooling. To this solution, triethylamine (4.25 mL, 30.7 mmol) was added, and trifluoroacetic acid (2.55 mL, 33.3 mmol) was dropwise added over a period of 20 minutes. Subsequently, methanesulfonyl chloride (2.40 mL, 31.0 mmol) was added, then the temperature was returned to room temperature from ice cooling, and the resulting solution was refluxed for 3 hours in a stream of nitrogen while heating it in an oil bath at 120°C. Then, the solution was cooled down to room temperature, and by the HPLC analysis, the yield proved to be 94%.

### Comparative Example 1

### (Method including reacting trifluoroacetic acid with methanesulfonyl chloride in a solvent first and then adding 2,3-diaminomaleonitrile to react them; solvent: N-methylpyrrolidone)

In N-methylpyrrolidone (29.4 ml), trifluoroacetic acid (0.45 ml, 0.67 g, 5.88 mmol), triethylamine (0.75 ml, 0.54 g, 5.38 mmol) and methanesulfonyl chloride (0.42 ml, 0.62 g, 5.42 mmol) were dissolved, the resulting solution was stirred and aged for 2 hours at room temperature, then 2,3-diaminomaleonitrile (0.53 g, 4.90 mmol) was added at the same temperature, and thereafter, they were continuously stirred for 18 hours while heating them in an oil bath at 129°C. Then, the resulting solution was cooled to room temperature and analyzed by HPLC. The production ratio of 4,5-dicyano-2-(trifluoromethyl)imidazole was 55%.

### Comparative Example 2

### (Method including reacting trifluoroacetic acid with methanesulfonyl chloride in a solvent first and then adding 2,3-diaminomaleonitrile to react them; solvent: acetonitrile)

Together with methanesulfonyl chloride (0.42 ml, 0.62 g, 5.42 mmol) having been dissolved in acetonitrile (4.9 ml), trifluoroacetic acid (0.45 ml, 0.67 g, 5.88 mmol) and triethylamine (0.75 ml, 0.54 g, 5.38 mmol) were stirred and aged for 2 hours at room temperature, then 2,3-diaminomaleonitrile (0.53 g, 4.90 mmol) having been dissolved in acetonitrile (19.6 ml) was added at the same temperature while stirring, and the resulting solution was refluxed while continuously stirring it for 18 hours and heating it in an oil bath at 82°C. Then, the solution was cooled to room temperature and analyzed by HPLC. The production ratio of 4,5-dicyano-2-(trifluoromethyl)imidazole was 62%.

### Comparative Example 3

### (Method including using isobutyl chloroformate instead of methanesulfonyl chloride and performing reaction in the same manner as in the present invention; solvent: N-methylpyrrolidone)

To N-methylpyrrolidone (29.4 ml), trifluoroacetic acid (0.45 ml, 0.67 g, 5.88 mmol), triethylamine (0.75 ml, 0.54 g, 5.38 mmol) and 2,3-diaminomaleonitrile (0.53 g, 4.90 mmol) were added, and immediately thereafter, heating was started in an oil bath at 129°C. Then, isobutyl chloroformate (0.71 ml, 0.74 g, 5.38 mmol) was added, and the resulting solution was continuously stirred for 18 hours, thereafter cooled to room temperature and analyzed by HPLC. The production ratio of 4,5-dicyano-2-(trifluoromethyl)imidazole was 6%.

## Claims

1. A method for producing a 4,5-dicyano-2-(fluoroalkyl)imidazole of formula (i): 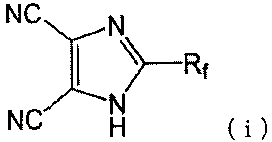
(wherein R_{f} is a C1-C10 fluoroalkyl group or a C3-C10 fluorocycloalkyl group), comprising reacting a compound of formula (ii):
R_{f}-COOH (ii)
(wherein R_{f} is the same as in formula (i)) or a salt thereof with a compound of formula (iii):
X-R (iii)
(wherein X is Cl, Br or I, and R is an unsubstituted or substituted C1-C6 alkylsulfonyl group or an unsubstituted or substituted phenylsulfonyl group) and diaminomaleonitrile in a solvent in the presence or absence of a base.

2. The method for producing the 4,5-dicyano-2-(fluoroalkyl)imidazole according to claim 1, wherein after the compound of formula (ii) and diaminomaleonitrile are added, the compound of formula (iii) is added.

3. The method for producing the 4,5-dicyano-2-(fluoroalkyl)imidazole according to claim 1 or 2, wherein the compound of formula (ii) is trifluoroacetic acid.

4. The method for producing the 4,5-dicyano-2-(fluoroalkyl)imidazole according to any one of claims 1 to 3, wherein the compound of formula (iii) is methanesulfonyl chloride.
